# EUROPEAN PATENT SPECIFICATION

(11) **EP 1 275 391 B1**
(45) Date of publication and mention of the grant of the patent: **30.03.2011**
(21) Application number: 02016237.6
(22) Date of filing: 07.06.1996
(51) Int. Cl.: A61K 31/415, A61K 9/00, A61K 9/20, A61K 31/5415

(54) **Pharmaceutical compositions containing irbesartan and a diuretic**
Arzneizubereitung enthaltend Irbesartan und ein Diuretikum
Composition pharmaceutique contenant de l'irbesartan et un diurétique

(30) Priority: 07.06.1995 US 472618
(43) Date of publication of application: 15.01.2003
(62) Divisional of application: 96304291.6
(73) Proprietor: Sanofi-Aventis, 75013 Paris (FR)
(72) Inventor: Ku, Cathy C., Martinsville, NJ 08836 (US); Sprockel, Omar L., Bridgewater, NJ 08807 (US); Rubitski, Beth A., Bedminster, NJ 07921 (US); Desai, Divyakant S., Robbinsville, NJ 08691 (US)
(74) Representative: Thouret-Lemaitre, Elisabeth

(56) References cited:
- EP-A- 0 475 898
- EP-A- 0 629 408
- EP-A- 0 708 103
- WO-A-91/14679
- WO-A1-94/09778
- US-A- 5 270 317
- US-A- 5 541 209

## Description

The present invention relates to pharmaceutical compositions containing irbesartan as disclosed in claim 1, preferably in the form of a tablet. The present invention also relates to tablets prepared from these compositions.

Irbesartan, 2-n-butyl-4-spirocyclopentane-1-[(2'-(tetrazol-5-yl)biphenyl-4-yl) methyl]-2-imidazolin-5-one, is a potent, long-acting angiotensin II receptor antagonist which is particularly useful in the treatment of cardiovascular ailments such as hypertension and heart failure. Irbesartan has the following structure: 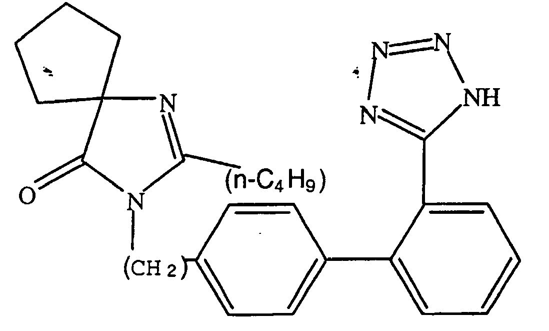 and is described in Bernhart et al., U.S. Patent No. 5,270,317. Preferred pharmaceutical compositions of this drug contain, as active ingredients, irbesartan in combination with a diuretic such as hydrochlorothiazide.

Irbesartan may be administered in dosages containing a substantial quantity of the active agent (e.g., 75 - 300 mg). Certain physical properties of the drug present a challenge in developing formulations suitable for preparing a tablet having both a substantial quantity of active agent and a small enough tablet mass to allow ease of swallowing.

Irbesartan is, for example, a fluffy material, with relatively low bulk and tap densities. These properties make it difficult to formulate a large amount of the drug into a small tablet with uniformity of weight, hardness, and other desirable tablet properties. In addition, irbesartan has certain undesirable flow characteristics, for example, is sticky and can adhere to surfaces such as tablet punch faces and dies, causing problems in tableting, especially on a high speed tablet press. The low aqueous solubility of irbesartan also presents a challenge, since, to keep the tablet mass small, only limited amounts of excipients may be added to facilitate wetting, disintegration, and ultimately, rapid and complete drug release. The addition of a diuretic such as hydrochlorothiazide, which is also a fluffy material exhibiting poor flow and low aqueous solubility, can further contribute to tableting problems.

Thus, there is a need in the art for pharmaceutical compositions containing irbesartan in combination with a diuretic, which have good properties for tablet formation, and yet which contain a low mass of excipients so that small, easily swallowed tablets with a high content of active agent may be prepared.

WO-A-91 14679 (of which US 5,270,317 above cited is a continuation-in-part) discloses heterocyclic N-substituted derivatives, namely irbesartan (claim), to be used in particular against hypertension and heart failure.

EP-A-0475895 discloses a family of azacyclic compounds having Angiotensin-II antagonist properties and pharmaceutical compositions containing them.

WO 94/09778 discloses pharmaceutical formulations comprising as active ingredients a diuretic and an A-II antagonist. Compositions A, B and C of example 1 comprise respectively losartan and hydrochlorothiazide.

US 5,270,317 describes pharmaceutical compositions containing N-substituted heterocyclic derivatives antagonizing the action of angiotensin II and optionally a further active principle, such as a diuretic.

At least in its preferred forms, the present invention provides pharmaceutical compositions containing irbesartan in combination with a diuretic, which (1) have a minimal mass of added excipients, thereby allowing preparation of small, easily swallowed tablets which enhance patient acceptance and compliance, and yet which (2) have excellent properties for tablet formation, and (3) provide tablets with excellent wetting, disintegration, and ultimately, rapid and complete drug release properties.

In particular, the present invention provides pharmaceutical compositions, especially suitable for forming tablets, comprising from 50% by weight irbesartan or pharmaceutically acceptable salts thereof, and pharmaceutically acceptable excipients, wherein a tablet formed from said composition has a dissolution performance such that 80% or greater, preferably 85% or greater, of the irbesartan or salts thereof contained in said tablet dissolve within 30 minutes. The present compositions also comprise a diuretic, wherein the combined amount of irbesartan and diuretic does not exceed 85%.

The present compositions may contain up to 85% w/w irbesartan and diuretic, and yet can be employed in the reproducible manufacture of tablets on a large scale. The present compositions can, for example, be compressed on high speed tableting equipment (especially, a high speed tablet press) to form tablets which are uniform in both weight and content and which exhibit desirable physical properties, including elegant appearance, low friability, and fast disintegration time. Tablets prepared from the present compositions are capable of releasing the active component(s), by dissolution, in a fast and reproducible manner.

Unless otherwise indicated, mention of irbesartan herein also includes pharmaceutically acceptable salts thereof.

The present invention is described in further detail as follows. The components employed in the compositions of the present invention should be pharmaceutically acceptable, particularly as described in the National Formulary (NF) or United States Pharmacopeia (USP).

The "dissolution performance" of a tablet, as used herein with respect to irbesartan, refers to the weight % of irbesartan, based on the total weight of irbesartan contained in the tablet which dissolves within 30 minutes under the following conditions: using a tablet having a total weight of from 150 to 600 mg and a USP Apparatus 2, placing the tablet in 1000 mL of 0.1 N hydrochloric acid at 37°C, with a paddle speed of 50 rpm, and measuring the amount of irbesartan dissolved (especially, using HPLC, wavelength 272 nm) at 30 minutes. (If desired, the progress of dissolution may also be monitored at various time points.)

The "dissolution performance" of a tablet, as used herein with respect to a diuretic (preferably, hydrochlorothiazide), refers to the weight % of diuretic, based on the total weight of diuretic contained in the tablet, which dissolves within 30 minutes under the conditions described above for irbesartan dissolution. The dissolution performance for the diuretic preferably meets the USP dissolution specification for the diuretic (for hydrochlorothiazide, greater than 60% dissolution at 30 minutes). The dissolution performance of a tablet containing hydrochlorothiazide is most preferably such that 90% or greater of the hydrochlorothiazide is dissolved at 30 minutes.

The "diuretic" employed in a composition of the present invention is hydrochlorothiazide.

The "diluent" employed in a composition of the present invention is lactose hydrous.

The "binder" employed in a composition of the present invention may be one or more compounds which are capable of facilitating granulation of the irbesartan and/or diuretic into larger, denser, and/or more free-flowing particles. A preferred binder is pregelatinized starch.

The "disintegrant" employed in a composition of the present invention may be one or more compounds which are capable of facilitating the break up of a tablet prepared from the composition when placed in contact with an aqueous medium. Preferred disintegrants are microcrystalline cellulose or croscarmellose sodium (cross-linked polymer of carboxymethylcellulose sodium) or pregelatinized starch.

The "antiadherent" employed in a composition of the present invention may be one or more compounds which are capable of reducing the stickiness of the formulation, for example, preventing adherence to metal surfaces. A preferred antiadherent is silicon dioxide.

The "lubricant" employed in a composition of the present invention may be one or more compounds which are capable of preventing tableting problems, such as those relating to the release of a tablet prepared from the composition from the apparatus on which it is formed, for example, preventing adherence to the face of the upper punch (picking) or lower punch (sticking) of a tableting apparatus. A preferred lubricant is magnesium stearate.

The "coloring agent" (or "colorant") employed in a composition of the present invention may be one or more compounds which impart a desired color to a tablet prepared from the composition. Addition of a coloring agent may be used, for example, so that tablets of different potencies may be easily distinguished. Preferred coloring agents are ferric oxides, which are universally accepted.

As can be seen from the above, a single compound may perform two or more functions. Calculation of weight percent is preferably on the basis of the primary function of a compound in a given composition. The present compositions preferably consist essentially of, most preferably, consist of the above-described components.

### Methods of Manufacture

Tablets may be prepared from the present compositions by any suitable method for forming tablets. Preferably, tablets are prepared from the present compositions by a wet granulation process. An exemplary such method comprises the following steps:
(a) preparing an intragranular composition by:
   (i) mixing the irbesartan, diuretic, a portion of the diluent (preferably, from 5 to 80% by weight of the total diluent), a portion of the disintegrant (preferably, from 50 to 80% by weight of the total disintegrant), the binder, and, optionally, a portion of the antiadherent (preferably, from 50 to 80% by weight of the total antiadherent), to form a powder blend and, optionally, sizing the blend (e.g., milling the blend to break up aggregates);
   (ii) re-mixing the blend;
   (iii) granulating the blend with a granulating fluid, preferably water, to form granules (e.g., using a high shear mixer/granulator);
   (iv) drying the granules (e.g., in an oven or, preferably, in a fluid bed dryer); and
   (v) sizing the dried granules (e.g., by milling or screening);
(b) preparing a mixture of the sized granules of step (a)(v) with an extragranular composition by:
   (i) mixing the remainder of the diluent, the remainder of the disintegrant, the antiadherent or the remainder of the antiadherent, and, optionally, the coloring agent, where one or more of these may be pre-blended, sized (e.g., milled to break up aggregates) and re-mixed prior to this step, with the sized granules from step (a)(v) to form a granule blend; and
   (ii) mixing the lubricant with the granule blend; and
(c) compressing the mixture from step (b)(ii) to form tablets (for example, employing a tablet press).

The solid starting materials of the present compositions are preferably screened prior to use. The weight ratio of water (preferably, purified water, USP or water for injection, USP) to solids employed in step (a)(iii) is preferably within the range of from 0.25:1 to 0.6:1.

The tablets may, optionally, be finished or coated such as by methods known in the art.

The tablets prepared from the compositions of the present invention preferably contain (per tablet) from 25 to 300 mg of irbesartan, most preferably from 75 to 300 mg of irbesartan and, for the combined tablets, an additional amount of from 1 to 25 mg of diuretic, most preferably from 6.25 to 25 mg of hydrochlorothiazide. The total weight of the tablets prepared is preferably from 50 to 600 mg. In addition to tablets, the compositions of the present invention may be used to prepare beads, granules for dispersion or capsules, the latter, for example, filled with powder or the aforementioned beads or granules. Methods such as those well known in the art may be used to prepare these dosage forms.

The compositions and tablets of the present invention may be used to treat or prevent disorders such as those described in U.S. Patent No. 5,270,317. Such disorders include cardiovascular disorders, for example, hypertension or heart failure, venous insufficiency, as well as glaucoma, diabetic retinopathy, renal insufficiency and various complaints of the central nervous system. The present compositions or tablets are preferably administered orally, in an effective amount, to a mammalian (especially, human) subject to treat or prevent the aforementioned disorders. For human subjects, preferred dosages of from 75 mg to 300 mg of irbesartan (alone or in combination with a diuretic) may be administered, for example, 1 to 2 times per day.

### EXAMPLE 1

### PREPARATION OF COMBINATION TABLETS: IRBESARTAN AND HYDROCHLOROTHIAZIDE

Tablets containing a combination of irbesartan and hydrochlorothiazide were prepared in two potencies from the composition of the present invention described in the following Table 1: (1) 75 mg irbesartan/12.5 mg hydrochlorothiazide with a total weight of 150 mg per tablet; and (2) 150 mg irbesartan/12.5 mg hydrochlorothiazide with a total weight of 300 mg per tablet.

**TABLE 1**

| Ingredient | Amount (% w/w) in 75 mg/12.5 mg Tablets | Amount (% w/w) in 150 mg/12.5 mg Tablets |
|---|---|---|
| **INTRAGRANULAR** | | |
| irbesartan | 50.00 | 50.00 |
| hydrochlorothiazide, USP | 8.33 | 4.17 |
| lactose hydrous NF (diluent) | 4.72 | 8.88 |
| pregelatinized starch, NF (binder) | 15.00 | 15.00 |
| croscarmellose sodium, NF (disintegrant) | 4.00 | 4.00 |

| **EXTRAGRANULAR** | | |
|---|---|---|
| microcrystalline cellulose, NF (diluent) | 15.00 | 15.00 |
| croscarmellose sodium, NF (disintegrant) | 1.00 | 1.00 |
| silicon dioxide, NF (antiadherent) | 0.75 | 0.75 |
| ferric oxide, NF, red (colorant) | 0.10 | 0.10 |
| ferric oxide, NF, yellow (colorant) | 0.10 | 0.10 |
| magnesium stearate, NF | 1.00 | 1.00 |
| **TOTAL** | 100.00 | 100.00 |

Tablets having the above compositions were prepared using a wet granulation process as follows. The irbesartan and hydrochlorothiazide drug substances, the lactose hydrous, the pregelatinized starch, and a portion (4/5 of the total amount) of the croscarmellose sodium were weighed out and mixed. This powder blend was then milled to break up aggregates of the drug(s). The milled powder blend was then mixed again, followed by granulation with water (in an amount which was 55% of the weight of total solids), in a mixer/granulator. The wet granules were then dried in drying equipment (tray or fluid bed dryer) until the loss on drying (LOD) was 2% or less, followed by milling of the dried granules.

A color blend was made by mixing the ferric oxides with a portion (1/3 of the total amount) of the microcrystalline cellulose, milling the color blend, then mixing again. The remaining microcrystalline cellulose, the remaining croscarmellose sodium, the color blend, and the silicon dioxide were then weighed, screened, and mixed in a mixer with the dried, milled granules. In a final step, the magnesium stearate was weighed, screened and mixed with the above granule blend. This final blend was then compressed into tablets using a suitable tablet press.

### Tablets Prepared

For the irbesartan/hydrochlorothiazide 75 mg/12.5 mg tablets, the tablet weight was 150 mg and the tablet hardness was 10 - 14 SCU (Strong Cobb Units). For the 150 mg/12.5 mg potency tablets, the tablet weight was 300 mg and the tablet hardness was 14 - 18 SCU. For both potency tablets, the friability was less than 0.5%, the disintegration time was under 7 minutes, and the coefficient of variation for tablet weight was under 2%. In addition, the dissolution of these tablets meets the specification for irbesartan dissolution of 85% or greater in 30 minutes and easily meets the USP dissolution specification for hydrochlorothiazide of 60% in 30 minutes.

The tablets of this formulation were found to have good stability. Under certain conditions, hydrochlorothiazide can hydrolyze to form, as by-products, a free amine degradant and formaldehyde (D.S. Desai et al., International Journal of Pharmaceutics, 107(2), 141-47 (1994)). The selection of excipients can impact the stability of hydrochlorothiazide. The use of pregelatinized starch as a binder in the present compositions was found to impart greater stability to hydrochlorothiazide than, for example, povidone (which resulted in the formation of quantities of the free amine degradant). Poloxamer was also found to increase the degradation of hydrochlorothiazide, and therefore, while employed as a preferred component in compositions without hydrochlorothiazide, poloxamer is not a preferred component for the irbesartan/hydrochlorothiazide compositions of the present invention. The aforementioned preferred compositions of the present invention containing irbesartan and hydrochlorothiazide are thus further advantageous since the excipients employed therein minimize or eliminate hydrochlorothiazide degradation.

### EXAMPLE 2: (REFERENCE EXAMPLES)

### PREPARATION OF COMBINATION TABLETS: IRBESARTAN AND HYDROCHLOROTHIAZIDE; ALTERNATIVE FORMULATIONS

Tablets were prepared having the compositions 2(A), 2(B), 2(C) or 2(D) of the following Table 2 by methods analogous to those of Example 1.

**TABLE 2**

| Ingredient | 2(A) mg per tablet (% w/w) | 2(B) mg per tablet (% w/w) | 2(C) mg per tablet (% w/w) | 2(D) mg per tablet (% w/w) |
|---|---|---|---|---|
| **INTRAGRANULAR** | | | | |
| irbesartan (active drug) | 750 (50) | 75.0 (50) | 150.0 (50) | 150.0 (50) |
| hydrochlorothiazide (diuretic, active drug) | 12.5 (8.33) | 12.5 (8.33) | 12.5 (4.17) | 12.5 (4.17) |
| lactose hydrous, NF (diluent) | 2.575 (1.72) | 17.575 (11.72) | 17.65 (5.88) | 47.65 (15.88) |
| pregelatinized starch, NF (binder) | 22.5 (15) | --- | 45.0 (15) | --- |
| povidone K-30, USP (binder) | --- | 7.5 (5) | --- | 15.0 (5) |
| croscarmellose sodium (disintegrant) | 6.0 (4) | 6.0 (4) | 12.0 (4) | 12.0 (4) |
| Pluronic F68, NF (poloxamer, surfactant) | 4.5 (3) | 4.5 (3) | 9.0 (3) | 9.0 (3) |

| **EXTRAGRANULAR** | | | | |
|---|---|---|---|---|
| microcrystalline cellulose, NF (diluent) | 22.5 (15) | 22.5 (15) | 45.0 (15) | 45.0 (15) |
| croscarmellose sodium (disintegrant) | 1.5 (1) | 1.5 (1) | 3.0 (1) | 3.0 (1) |
| ferric oxide, NF red (colorant) | 0.15 (0.1) | 0.15 (0.1) | 0.3 (0.1) | 0.3 (0.1) |
| ferric oxide, NF yellow (colorant) | 0.15 (0.1) | 0.15 (0.1) | 0.3 (0.1) | 0.3 (0.1) |
| silicon dioxide (antiadherent) | 1.125 (0.75) | 1.125 (0.75) | 2.25 (0.75) | 2.25 (0.75) |
| magnesium stearate (lubricant) | 1.5 (1) | 1.5 (1) | 3.0 (1) | 3.0 (1) |
| **TOTAL WEIGHT** | 150.0 (100) | 150.0 (100) | 300.0 (100) | 300.0 (100) |

In the following claims list of ingredients are intended to cover (a) either the ingredients in the actual composition as would be ascertained by analysis or (b) in the ingredients from which the composition is prepared (this allowing for any reaction which might take place between the ingredients).

## Claims

1. A pharmaceutical composition comprising, based on weight:
- 50% irbesartan;
- 8.33% hydrochlorothiazide and 4.72% lactose hydrous or 4,17% hydrochlorothiazide and 8.88% lactose hydrous;
- 15.0% pregelatinized starch;
- 5.0% croscarmellose sodium;
- 15% microcrystalline cellulose;
- 0.75% silicon dioxide;
- 1.0% magnesium stearate:
- 0.1% ferric oxide, red; and
- 0.1% ferric oxide, yellow,
wherein a tablet formed from said composition has a dissolution performance such that 80% or greater of the irbesartan or salt thereof contained in said tablet dissolves within 30 minutes under the following conditions: using a tablet having a total weight of from 150 me to 600 mg and a USP apparatus 2, placing the tablet in 1000 mL of 0.1 N hydrochloric acid at 37°C. with a paddle speed of 50 rpm, and measuring the amount of irbesartan dissolved.

2. A pharmaceutical composition according to claim 1, comprising, based on weight, 8.33% hydrochlorothiazide; and 4.72% lactose hydrous.

3. A pharmaceutical composition according to claim 1, comprising, based on weight, 4.17% hydrochlorothiazide; and 8.88% lactose hydrous.

4. A tablet formed from the composition of any preceded claim.

5. A tablet of claim 4, wherein the total weight of said tablet is from 50 to 600 mg.

6. A tablet formed from the composition of any one of claims 1 to 3 wherein said tablet is prepared by mixing an extragranular composition with granules comprising an antiadherent.

## Patentansprüche

1. Pharmazeutische Zusammensetzung, umfassend, bezogen auf das Gewicht:
- 50% Irbesartan;
- 8,33% Hydrochlorothiazid und 4,72% wasserhaltige Lactose oder 4,17% Hydrochlorothiazid und 8,88% wasserhaltige Lactose;
- 15,0% Quellstärke;
- 5,0% Natriumcroscarmellose;
- 15% mikrokristalline Cellulose;
- 0,75% Siliciumdioxid;
- 1,0% Magnesiumstearat;
- 0,1% Eisenoxidrot und
- 0,1% Eisenoxidgelb;
wobei eine aus der Zusammensetzung gebildete Tablette ein solches Auflösungsverhalten zeigt, daß 80% oder mehr des in der Tablette enthaltenen Irbesartans oder Salzes davon sich innerhalb von 30 Minuten lösen, und zwar unter den folgenden Bedingungen: Verwenden einer Tablette mit einem Gesamtgewicht von 150 mg bis 600 mg und und einer USP-Apparatur 2, Einbringen der Tablette in 1000 mL 0,1 N Salzsäure bei 37°C bei einer Blattgeschwindigkeit von 50 U/min und Messen der Menge von gelöstem Irbesartan.

2. Pharmazeutische, Zusammensetzung nach Anspruch 1, umfassend, bezogen auf das Gewicht, 8,33% Hydrochlorothiazid und 4,72% wasserhaltige Lactose.

3. Pharmazeutische Zusammensetzung nach Anspruch 1, umfassend, bezogen auf das Gewicht, 4,17% Hydrochlorothiazid und 8,88% wasserhaltige Lactose.

4. Tablette, geformt aus der Zusammensetzung nach einem der vorhergehenden Ansprüche.

5. Tablette nach Anspruch 4, wobei das Gesamtgewicht der Tablette 50 bis 600 mg beträgt.

6. Tablette, geformt aus der Zusammensetzung nach einem der Ansprüche 1 bis 3, wobei die Tablette durch Mischen einer extragranulären Zusammensetzung mit Körnchen, die ein Antihaftmittel enthalten, hergestellt worden ist.

## Revendications

1. Composition pharmaceutique comprenant, sur la base du poids :
- 50 % d'irbesartan ;
- 8,33 % d'hydrochlorothiazide et 4,72 % de lactose hydraté ou 4,17 % d'hydrochlorothiazide et 8,88 % de lactose hydraté ;
- 15,0 % d'amidon prégélatinisé ;
- 5,0 % de croscarmellose sodique ;
- 15 % de cellulose microcristalline ;
- 0,75 % de dioxyde de silicium ;
- 1,0 % de stéarate de magnésium ;
- 0,1 % d'oxyde ferrique, rouge ; et
- 0,1 % d'oxyde ferrique, jaune,
un comprimé formé à partir de ladite composition ayant une performance de dissolution telle que 80 % ou plus de l'irbesartan ou de sel de celui-ci contenus dans ledit comprimé se dissolvent en 30 minutes dans les conditions suivantes : en utilisant un comprimé ayant un poids total de 150 mg à 600 mg et un appareil 2 d'USP, en plaçant le comprimé dans 1000 ml d'acide chlorhydrique 0,1 N à 37°C, avec une vitesse de pale de 50 t/min, et en mesurant la quantité d'irbesartan dissoute.

2. Composition pharmaceutique selon la revendication 1, comprenant, sur la base du poids, 8,33 % d'hydrochlorothiazide et 4,72 % de lactose hydraté.

3. Composition pharmaceutique selon la revendication 1, comprenant, sur la base du poids, 4,17 % d'hydrochlorothiazide et 8,88 % de lactose hydraté.

4. Comprimé formé à partir de la composition de l'une quelconque des revendications précédentes.

5. Comprimé selon la revendication 4, **caractérisé en ce que** le poids total dudit comprimé est de 50 à 600 mg.

6. Comprimé formé à partir de la composition selon l'une quelconque des revendications 1 à 3, **caractérisé en ce que** ledit comprimé est préparé en mélangeant une composition extragranulaire avec des granulés comprenant un antiadhérent.
